# EUROPEAN PATENT SPECIFICATION

(11) **EP 3 253 386 B1**
(45) Date of publication and mention of the grant of the patent: **01.03.2023**
(21) Application number: 16746769.5
(22) Date of filing: 21.01.2016
(51) Int. Cl.: A61K 31/4439, A61K 31/437, A61K 31/4178, A61K 31/4196, C07D 401/14, C07D 403/14, C07D 471/04, A61P 35/02

(54) **PHARMACEUTICAL COMPOSITION FOR PREVENTING OR TREATING CHRONIC MYELOID LEUKEMIA AND METHOD USING THE SAME**
PHARMAZEUTISCHE ZUSAMMENSETZUNG ZUR PRÄVENTION ODER BEHANDLUNG VON CHRONISCHER MYELOIDLEUKÄMIE UND VERFAHREN ZUR VERWENDUNG DAVON
COMPOSITION PHARMACEUTIQUE POUR PRÉVENIR OU TRAITER LA LEUCÉMIE MYÉLOÏDE CHRONIQUE ET SON PROCÉDÉ D'UTILISATION

(30) Priority: 04.02.2015 JP 2015020709; 23.06.2015 KR 20150089091
(43) Date of publication of application: 13.12.2017
(73) Proprietor: Medpacto Inc., Seoul 06668 (KR)
(72) Inventor: HA, Il Ho, Gunpo-si Gyeonggi-do 15821 (KR); NAKA, Kazuhito, Hiroshima 734-0007 (JP); LEE, Lee Young, Suwon-si Gyeonggi-do 16226 (KR)
(74) Representative: V.O.
(86) International application number: PCT/KR2016/000647
(87) International publication number: WO 2016/126028

(56) References cited:
- WO-A2-2009/115572
- WO-A2-2012/002680
- US-A1- 2011 319 408
- US-A1- 2011 319 408
- TAREQ AL BAGHDADI ET AL: "Novel Combination Treatments Targeting Chronic Myeloid Leukemia Stem Cells", CLINICAL LYMPHOMA MYELOMA AND LEUKEMIA, vol. 12, no. 2, 1 April 2012 (2012-04-01), pages 94-105, XP055474298, ISSN: 2152-2650, DOI: 10.1016/j.clml.2011.10.003
- KAZUHITO NAKA ET AL: "TGF-&bgr;-FOXO signalling maintains leukaemia-initiating cells in chronic myeloid leukaemia", NATURE, vol. 463, no. 7281, 4 February 2010 (2010-02-04), pages 676-680, XP055331194, GB ISSN: 0028-0836, DOI: 10.1038/nature08734
- KAZUHITO NAKA ET AL: "Novel oral transforming growth factor-[beta] signaling inhibitor EW-7197 eradicates CML-initiating cells", CANCER SCIENCE, vol. 107, no. 2, 19 November 2015 (2015-11-19), pages 140-148, XP055474395, JP ISSN: 1347-9032, DOI: 10.1111/cas.12849
- JIN, C. H. ET AL.: 'Discovery of N-((4-([1,2,4]Triazolo[1,5-a]pyridin-6-yl) -5-(6-methylpyridin-2-yl)-1H-imidazol-2-yl) methyl)-2-fluoroaniline(EW-7197): A Highly Potent, Selective, and Orally Bioavailable Inhibitor of TGF-beta Type I Receptor Kinase as Cancer Immunotherapeutic/Antifibrotic Agent' JOURNAL OF MEDICINAL CHEMISTRY vol. 57, no. 10, 01 January 2014, pages 4213 - 4238, XP055475279 DOI: 10.1021/JM500115W
- SON, JI YEON ET AL.: 'EW-7197, a Novel ALK-5 Kinase Inhibitor, Potently Inhibits Breast to Lung Metastasis' MOLECULAR CANCER THERAPEUTICS vol. 13, no. 7, 01 July 2014, pages 1704 - 1716, XP055320188 DOI: 10.1158/1535-7163.MCT-13-0903

## Description

### TECHNICAL FIELD

The present disclosure relates to a pharmaceutical composition for use in a method of preventing or treating chronic myeloid leukemia.

### BACKGROUND ART

Chronic myeloid leukemia (CML) is an abnormal myeloproliferative disease derived from abnormal expansion of the clone of hematopoietic stem cells. CML is caused by a Philadelphia chromosome, which results from the translocation between chromosomes 9 and 22 (t(9;22)(q34;q11)). This chromosomal translocation results in the fusion between ABL gene on chromosome 9 and BCR gene on chromosome 22, and this BCR-ABL fusion gene produces a BCR-ABL fusion protein with abnormal tyrosine kinase activity. BCR-ABL tyrosine kinase induces abnormal cell division. Recently, tyrosine kinase inhibitor (TKI)-insensitive CML problematically occurs in CML patients after TKI therapy. Since TKI targets only actively dividing CML cells, it does not eliminate quiescent CML stem cells. For radical treatment of CML, therefore, there is a need to kill CML stem cells as well as CML cells.

US 2011/319408 A1 discloses TGF-beta receptor (ALK5) inhibitors, including TEW-7197, for use in a method of treating cancer, in particular leukemia. Tareq al Baghdadi et al. (Clinical Lymphoma, Myeloma and Leukemia, vo. 12, 1 April 2012) describes positive effects of adding a TGF-beta inhibitor to tyrosine kinase inhibitor treatment when treating CML and Kazuhito N et al. (Nature, vol. 463, no. 7281, 4 February 2010) discloses that inhibition of TGF-beta enhanced imatinib-induced cell death in the treatment of CML *in vitro* and *in vivo.*

Transforming growth factor (TGF)-β is a cytokine that modulates cell proliferation and differentiation, wound healing, extracellular matrix production, etc. TGF-β family belongs to TGF-β superfamily, and this TGF-β superfamily includes activins, inhibins, bone morphogenetic proteins, and anti-Mullerrian hormone. The tumor cells and the stromal cells within the tumors in late stages of various cancers generally overexpress TGF-β. TGF-β would lead to stimulation of angiogenesis and cell motility, suppression of the immune system, and increased interaction of tumor cells with the extracellular matrix. TGF-β receptor is a serine/threonine kinase receptor, and divided into TGF-β receptor 1, TGF-β receptor 2, and TGF-β receptor 3. Of them, TGF-β receptor 1 is also called activin A receptor type II-like kinase (ALK5).

Accordingly, for effective prevention or treatment of CML, there is a demand for a pharmaceutical composition capable of effectively inhibiting tyrosine kinase and TGF-β signaling pathway.

### DISCLOSURE

### TECHNICAL SOLUTION

Provided is a pharmaceutical composition for preventing or treating chronic myeloid leukemia, the composition including a compound having a TGF-β signaling pathway-inhibiting activity and a tyrosine kinase inhibitor.

Provided is a method of preventing or treating chronic myeloid leukemia, the method including administrating a compound having a TGF-β signaling pathway-inhibiting activity and a tyrosine kinase inhibitor to a subject.

### ADVANTAGEOUS EFFECTS

A pharmaceutical composition for use in a method of preventing or treating chronic myeloid leukemia.

### DESCRIPTION OF DRAWINGS

These and/or other aspects will become apparent and more readily appreciated from the following description of the embodiments, taken in conjunction with the accompanying drawings in which:

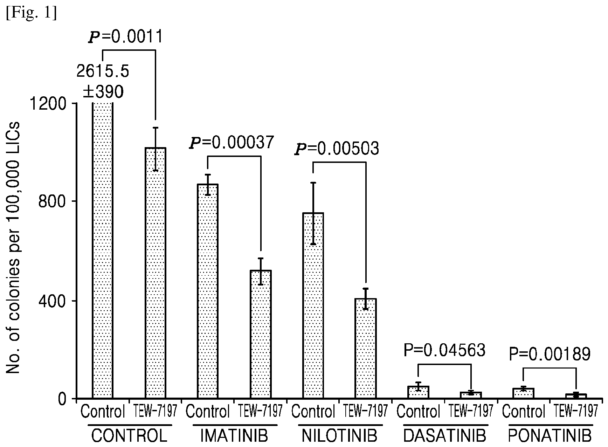

FIG. 1 is a graph showing colony formation of LT-CML stem cells cultured in the presence of imatinib, nilotinib, dasatinib, ponatinib, or TEW-7197 alone, or a combination thereof;

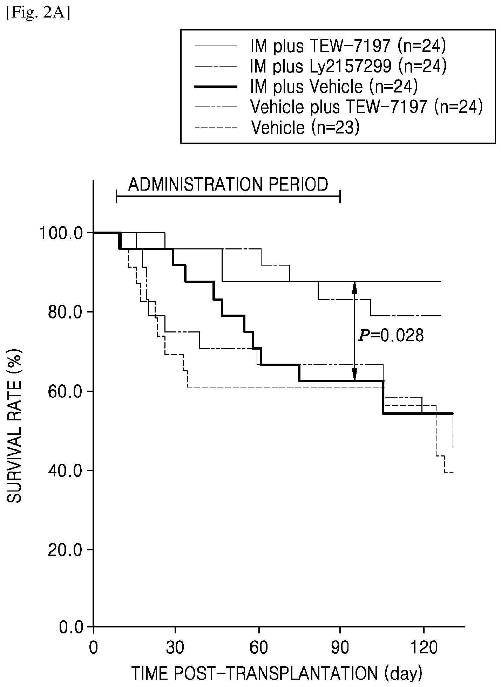

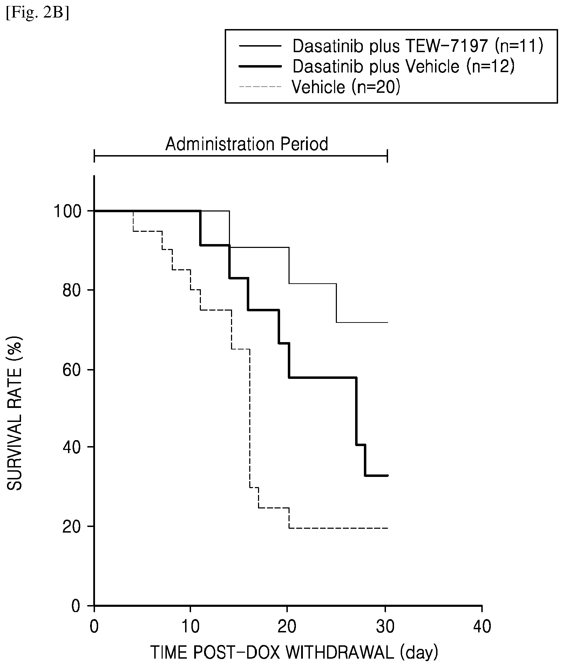

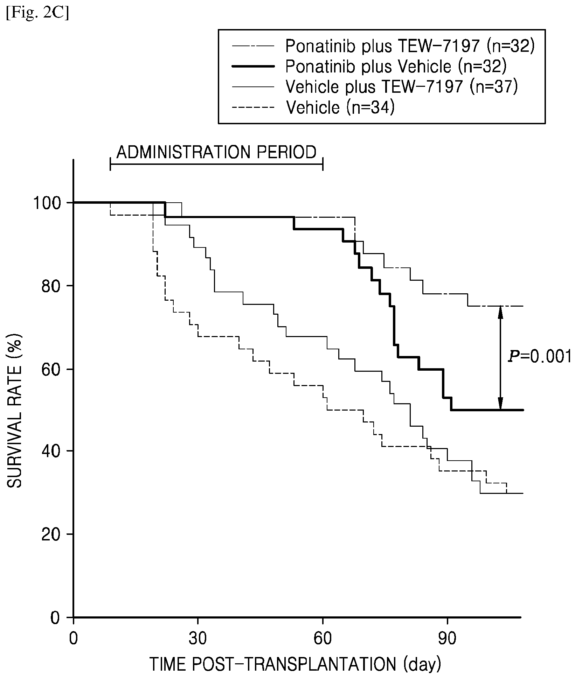

FIG. 2A is a graph showing survival rate (%) of TT-CML affected mouse according to drug administration, FIG. 2B is a graph showing survival rate (%) of tg-CMI, affected mouse over time after the end of doxycycline treatment, and FIG. 2C is a graph showing survival rate (%) of TKI-resistant T315I TT-CML affected mouse according to drug administration;

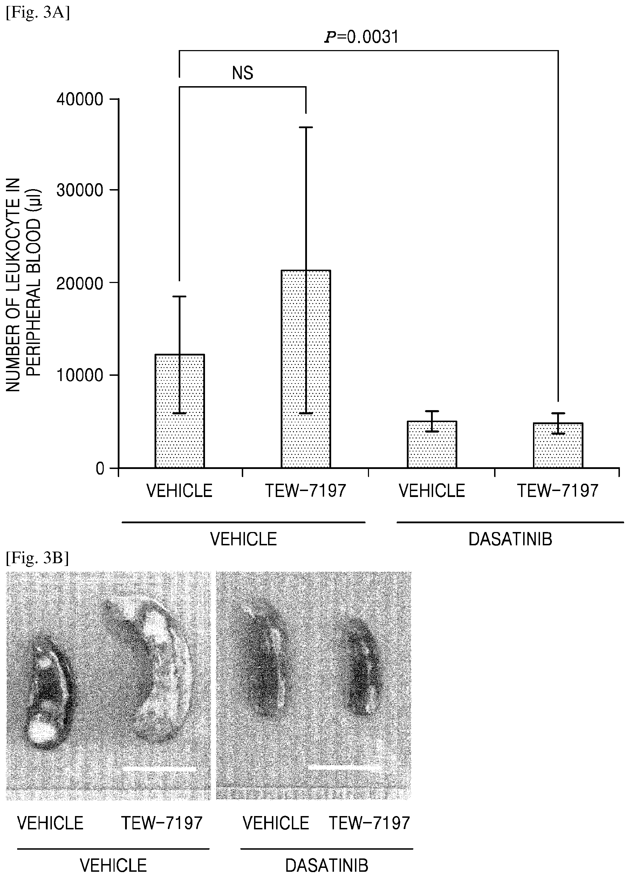

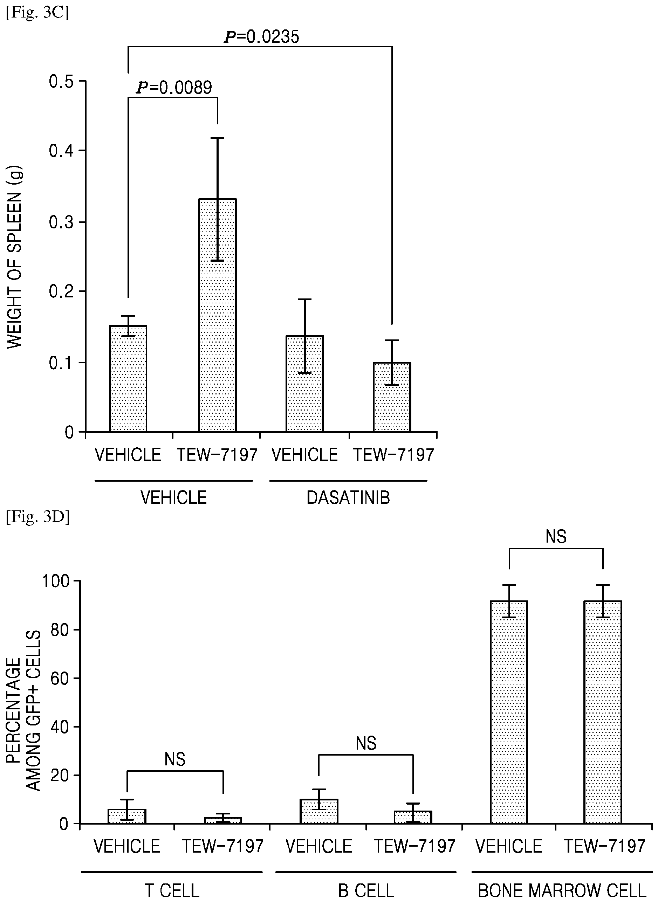

FIGS. 3A through 3C are a graph showing the number of leukocyte in the peripheral blood of the drug-administered TT-CML affected mouse, a photograph of the spleen thereof, and a graph showing the weight of the spleen thereof, respectively and FIG. 3D is a graph showing percentages (%) of T cell, B cell, and bone marrow cell among the total GFP/BCR-ABL⁺ cells in the peripheral blood of TEW-7197-administered TT-CML affected mouse;

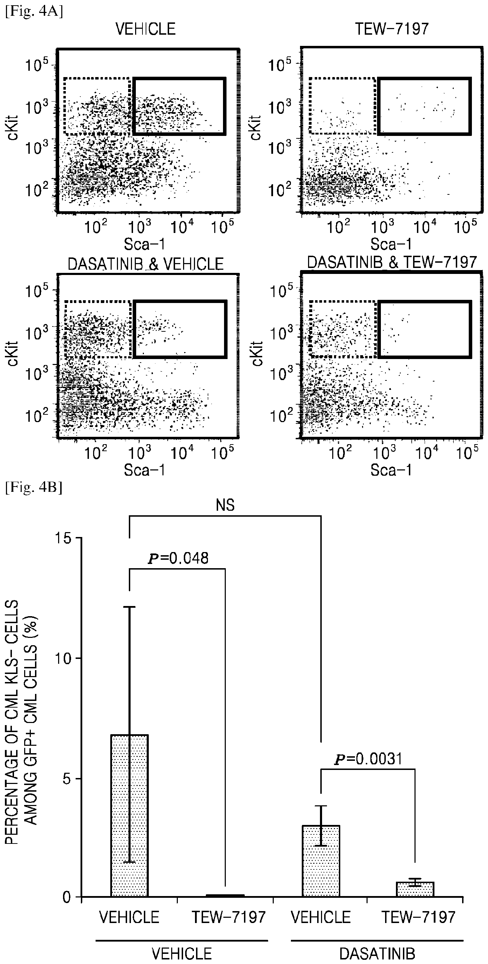

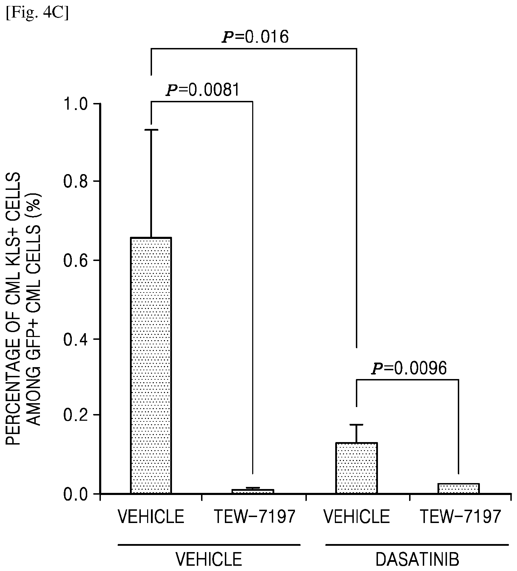

FIG. 4A is the flow cytometry result of GFP/BCR-ABL⁺ KLS⁺ cells (bold box) and KLS⁻cells (dotted box) in drug-administered TT-CML affected mouse, FIG. 4B is a graph showing the percentage (%) of CML KLS⁻ cells among GFP⁺-CML cells, and FIG. 4C is a graph showing the percentage (%) of CML KLS⁺ cells among GFP⁺-CML cells;

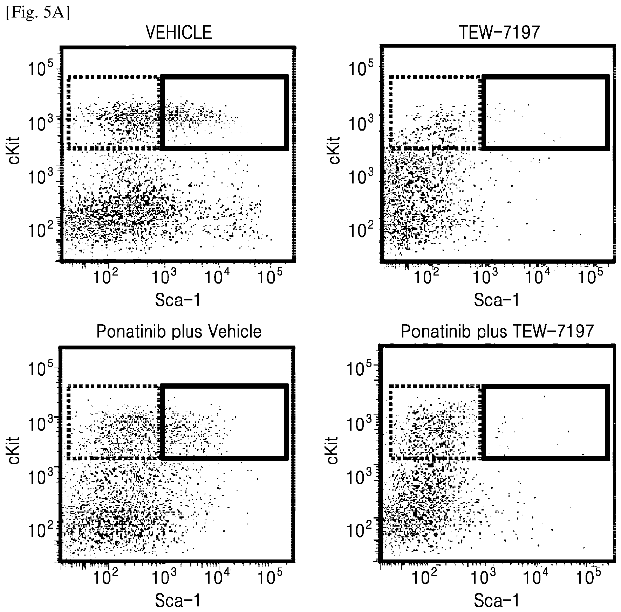

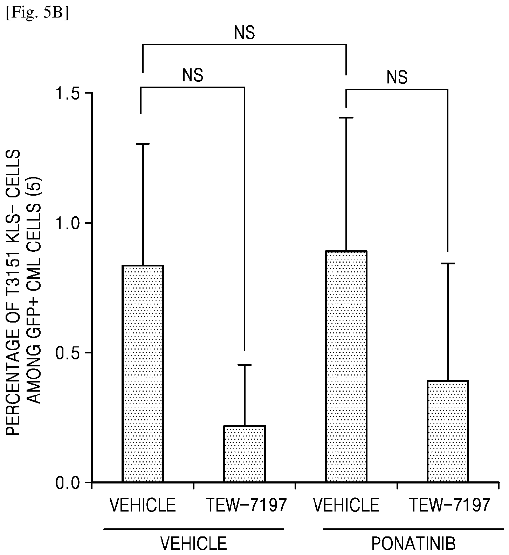

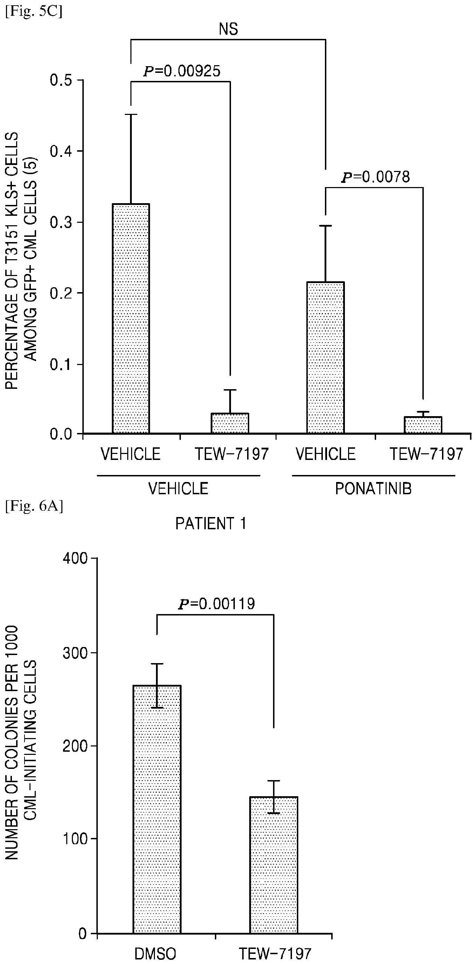

FIG. 5A is the flow cytometry result of T315I BCR-ABL-GFP⁺ KLS⁺ cells (bold box) and KLS⁻ cells (dotted box) in drug-administered TT-CML affected mouse, FIG. 5B is a graph showing the percentage (%) of T315I KLS⁻ cells among T315I BCR-ABL-GFP⁺ cells, and FIG. 5C is a graph showing the percentage (%) of T315I KLS⁺ cells among T315I BCR-ABL-GFP⁺ cells; and

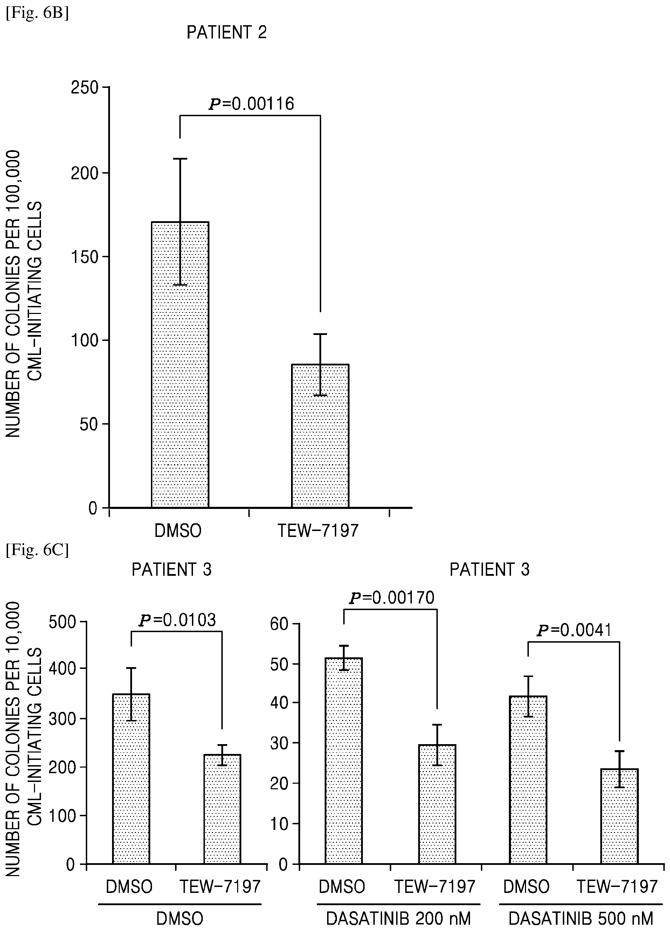

FIGS. 6A through 6C are graphs showing colony formation of human CML-initiating cells derived from three patients.

### MODE FOR INVENTION

An aspect provides a pharmaceutical composition for use in a method of preventing or treating chronic myeloid leukemia (CML), the composition including a compound of the following Chemical Formula I according to claims 1 to 6, a pharmaceutically acceptable salt, solvate, or stereoisomer thereof, or a combination thereof; and a tyrosine kinase inhibitor:

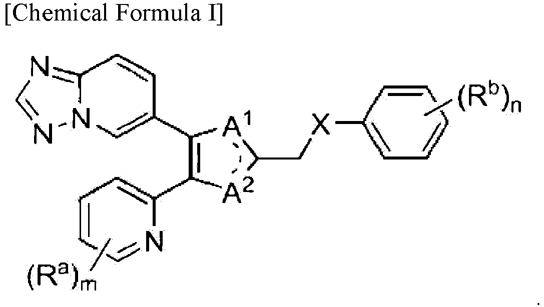

In the chemical formula I, R^{a} may be independently H, halo, C₁₋₆ alkyl, C₁₋₆ haloalkyl, C₃₋₆ cycloalkyl, OH, -O-C₁₋₆ alkyl, -O-C₁₋₆ haloalkyl, -O-C₃₋₆ cycloalkyl, NH₂, -NH-C₁₋₆ alkyl, -NH-C₁₋₆ haloalkyl, -NH-C₃₋₆ cycloalkyl, -S-C₁₋₆ alkyl, -S-C₁₋₆ haloalkyl, -S-C₃₋₆ cycloalkyl, CN, or NO₂.
m may be 0, 1, 2, 3 or 4.
Any one of A¹ and A² may be N and the other may be NR¹, in which R¹ is H, OH, C₁₋₆ alkyl, C₁₋₆ haloalkyl, or C₃₋₆ cycloalkyl.
X may be -NR²⁻, -O- or -S-, in which R² is H or C₁₋₃ alkyl.
R^{b} may be each independently H, halo, C₁₋₆ alkyl, C₁₋₆ haloalkyl, C₃₋₆ cycloalkyl, C₂₋₆ alkenyl, C₂₋₆ alkynyl, -(CH₂)_{q}-OR³, -(CH₂)_{q}-NR³R⁴, -(CH₂)_{q}-SR³, -(CH₂)_{q}-NO₂, -(CH₂)_{q}-CONHOH, -(CH₂)_{q}-CN, -(CH₂)_{q}-COR³, -(CH₂)_{q}-CO₂R³, -(CH₂)_{q}-CONR³R⁴, -(CH₂)_{q}- tetrazole, - (CH₂)_{q}-CH=CH-CN, -(CH₂)_{q}-CH=CH-CO₂R³, -(CH₂)_{q}-CH=CH-CONR³R⁴, -(CH₂)_{q}-CH=CH-tetrazole, -(CH₂)_{q}-NHCOR³, -(CH₂)_{q}-NHCO₂R³, -(CH₂)_{q}-CONHSO₂R³, -(CH₂)_{q}-NHSO₂R³, - (CH₂)_{q}-C=C-CN, -(CH₂)_{q}-C=C-CO₂R³, -(CH₂)_{q}-C=C-CONR³R⁴, -(CH₂)_{q}-C=C-tetrazole, -(CH₂)_{q}-SOR⁵, -(CH₂)q-SO₂R⁵, or -(CH₂)ᵣ-(OR³)₂, in which R³ and R⁴ are each independently H, C₁₋₆ alkyl, C₁₋₆ haloalkyl, or C₃₋₆ cycloalkyl, or taken together with the nitrogen atom bound thereto to form a mono-cyclic ring, for example, imidazole, pyrrolidine, piperidine, morpholine, piperazine and homopiperazine; R⁵ is C₁₋₆ alkyl, C₁₋₆ haloalkyl, or C₃₋₆ cycloalkyl; q is 0, 1, 2, 3, or 4; and r is 1, 2, 3, or 4.
n may be 0, 1, 2, 3, 4 or 5.

The alkyl group may be straight or branched. Examples of the alkyl group include methyl, methyl, ethyl, propyl, isopropyl, butyl, isobutyl, sec-butyl, tert-butyl, n-pentyl, and n-hexyl. The alkyl group may be substituted with one or more of alkoxy, cycloalkoxy, amino, nitro, carboxy, cyano, halo, hydroxyl, sulfo, or mercapto.

The cycloalkyl group is, for example, cyclopropyl, cyclobutyl, cyclopentyl, and cyclohexyl.

The halo is, for example, fluorine, chlorine, bromine, or iodine.

The alkenyl group may be straight or branched. The alkenyl group is, for example, vinyl, allyl, isoprenyl, 2-butenyl, and 2-hexenyl. The alkenyl group may be substituted with, for example, alkoxy, cycloalkoxy, amino, nitro, carboxy, cyano, halo, hydroxyl, sulfo, or mercapto.

The alkynyl group may be straight or branched. The alkynyl group is, for example, ethynyl, propargyl, and 2-butynyl. The alkynyl group may be substituted with, for example, alkoxy, cycloalkoxy, amino, nitro, carboxy, cyano, halo, hydroxyl, sulfo, or mercapto.

The compound of the present invention is a compound of the following Chemical Formula II:

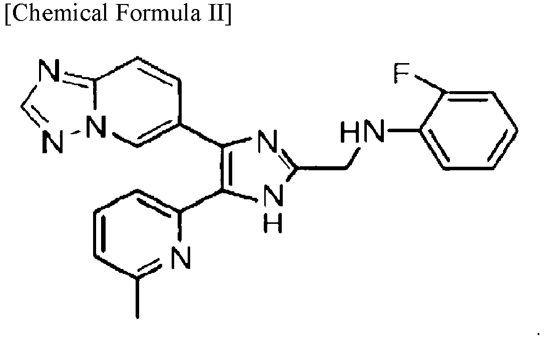

The compound of Chemical Formula II is N-((4-([1,2,4]triazolo[1,5-a]pyridin-6-yl)-5-(6-methylpyridin-2-yl)-1H-imidazol-2-yl)methyl)-2-fluoroaniline(called "TEW-7197").

The compound of Chemical Formula I, which is the compound of Chemical Formula II may selectively inhibit TGF-β receptor 1(ALK5) and/or activin receptor type-1B (ACVR1B, or ALK-4).

The pharmaceutically acceptable salt may be a salt that does not cause significant irritation to an organism to which the compound is administered and does not abrogate the biological activity and properties of the compound. The salt may be, for example, an inorganic acid salt, organic acid salt, or metal salt. The inorganic acid salt may be a salt of hydrochloric acid, bromic acid, phosphoric acid, sulfuric acid, or disulfuric acid. The organic acid salt may be a salt of mesylic acid, formic acid, acetic acid, propionic acid, lactic acid, oxalic acid, tartaric acid, malic acid, maleic acid, citric acid, fumaric acid, besylic acid, camsylic acid, edisylic acid, trichloroacetic acid, trifluoroacetic acid, benzoic acid, gluconic acid, methanesulfonic acid, glycolic acid, succinic acid, 4-toluenesulfonic acid, galacturonic acid, embonic acid, glutamic acid, ethanesulfonic acid, benzenesulfonic acid, p-toluenesulfonic acid, or aspartic acid. The metal salt may be a calcium salt, sodium salt, magnesium salt, strontium salt, or potassium salt.

The solvate may be a compound formed by attractive forces between solute and solvent molecules. The solvate may be, for example, a hydrate.

The stereoisomers refer to molecules that have the same molecular formula and connectivity of their atoms, but differ in spatial arrangement of atoms. The stereoisomers may be diastereomers or enantiomers of the compound of Chemical Formula II.

The term "tyrosine kinase (TK)" refers to a protein capable of transferring phosphate groups of ATP to tyrosine residues of proteins. Tyrosine kinase plays an important role in cell activity, for example, signal transduction regulating cell division. The tyrosine kinase may be Bcr-Abl tyrosine kinase. Bcr-Abl tyrosine kinase may be a protein that is produced from a BCR-ABL fusion gene between ABL gene on chromosome 9 and BCR gene on chromosome 22, resulting from the translocation between human chromosomes 9 and 22 (t(9;22)(q34;q11)).

The term "tyrosine kinase inhibitor (TKI)" refers to a drug inhibiting tyrosine kinase. The tyrosine kinase inhibitor may be a Bcr-Abl tyrosine kinase inhibitor. The tyrosine kinase inhibitor is imatinib, dasatinib (brand name: sprycel), nilotinib (brand name: Tasigna), bosutinib, ponatinib, or a combination thereof. Imatinib may be imatinib mesylate (brand name: Gleevec or Glivec).

The CML may be tyrosine kinase inhibitor-resistant. The tyrosine kinase inhibitor resistance includes tyrosine kinase inhibitor resistance acquired during CML treatment as well as initial resistance to tyrosine kinase inhibitors. The tyrosine kinase inhibitor resistance may be caused by Bcr-Abl dependent and independent mechanisms. Bcr-Abl dependent mechanism may include amplification of Bcr-Abl gene, mutation of Bcr-Abl gene, mutation of tyrosine kinase binding site, or a combination thereof. The mutation of Bcr-Abl gene may be phosphate binding loop (p-loop) mutation (e.g., G250E, Q252H, Y253F, Y253H, E255K, and E255V). The mutation of tyrosine kinase binding site may be, for example, T315I, T315A, F317L, and F317V. For example, tyrosine kinase inhibitor-resistance may be caused by a BCR-ABL fusion protein (T315I) in which a tyrosine residue is mutated by an isoleucine residue at position 315 from the N-terminus. Bcr-Abl independent mechanism may include drug efflux caused by P-glycoproteins, drug import by organic cation transporter 1 (OCT 1), and activation of alternative signaling pathway, for example, Src family kinase. Since TEW-7197 inhibits CML stem cells, it may have a prophylactic or therapeutic effect on any tyrosine kinase inhibitor-resistant CML having different mechanisms.

As used herein, the term "prevention" means all of the actions by which the occurrence of chronic myeloid leukemia is restrained or retarded by administration of the pharmaceutical composition, and the term "treatment" means all of the actions by which the symptoms of chronic myeloid leukemia have taken a turn for the better or been modified favorably by administration of the pharmaceutical composition.

The pharmaceutical composition may include a pharmaceutically acceptable carrier. The carrier includes an excipient, a diluent or an auxiliary agent. The carrier may be selected from the group consisting of lactose, dextrose, sucrose, sorbitol, mannitol, xylitol, erythritol, maltitol, starch, acacia rubber, alginate, gelatin, calcium phosphate, calcium silicate, cellulose, methylcellulose, polyvinylpyrrolidone, water, physiological saline, a buffer such as PBS, methylhydroxybenzoate, propylhydroxybenzoate, talc, magnesium stearate, and mineral oils. The composition may include a filler, an anti-coagulating agent, a lubricant, a humectant, a flavor, an emulsifier, an antiseptic, etc.

The pharmaceutical composition may be prepared into any formulation by a general method. The composition may be prepared into, for example, an oral formulation (e.g., powder, tablet, capsule, syrup, pill, granule) or a parenteral formulation (e.g., injectable formulation). Further, the composition may be prepared into a topical or systemic formulation.

The pharmaceutical composition may be prepared as a single composition or individual compositions.

The pharmaceutical composition includes the compound of Chemical Formula II, a pharmaceutically acceptable salt, solvate, or stereoisomer thereof, or a combination thereof; and a tyrosine kinase inhibitor, which is imatinib, dasatinib, nilotinib, bosutinib, ponatinib, or a combination thereof, in an effective amount. The term "effective amount" refers to an amount sufficient to exhibit a prophylactic or therapeutic effect when administered to a subject in need of prevention or treatment. The effective amount may be properly selected by those skilled in the art according to a cell or a subject to be selected. The effective amount may be determined depending on the severity of disease, a patient's age, body weight, health conditions, gender, and drug sensitivity, administration time, administration route, excretion rate, treatment period, a drug blended with or co-administered with the composition, and other factors well known in the medical field. The effective amount of the compound of Chemical Formula II, a pharmaceutically acceptable salt, solvate, or stereoisomer thereof, or a combination thereof may be about 0.5 *µ*g to about 2 g, about 1 *µ*g to about 1 g, about 10 *µ*g to about 500 mg, about 100 *µ*g to about 100 mg, about 1 mg to about 90 mg, about 5 mg to about 80 mg, about 10 mg to about 70 mg, about 15 mg to about 60 mg, or about 20 mg to about 50 mg, based on the pharmaceutical composition. The effective amount of the tyrosine kinase inhibitor may be about 0.5 *µ*g to about 2 g, about 1 *µ*g to about 1 g, about 10 *µ*g to about 500 mg, about 100 *µ*g to about 100 mg, about 1 mg to about 50 mg, about 5 mg to about 40 mg, or about 10 mg to about 30 mg, based on the pharmaceutical composition.

The administration dose of the pharmaceutical composition may be, for example, in the range from about 0.001 mg/kg to about 100 mg/kg, about 0.01 mg/kg to about 10 mg/kg, or about 0.1 mg/kg to about 1 mg/kg per adult. The administration may be performed once a day, several times a day, twice or three times a week, once to four times a month, or once or twelve times a year.

The compound of Chemical Formula II, the pharmaceutically acceptable salt, solvate, or stereoisomer thereof, the tyrosine kinase, the tyrosine kinase inhibitor, the chronic myeloid leukemia, the prevention and treatment are the same as described above.

The subject may be a mammal, for example, human, cow, horse, pig, dog, sheep, goat or cat. The subject may be a subject having chronic myeloid leukemia or at risk of having chronic myeloid leukemia.

The compound, a pharmaceutically acceptable salt, solvate, or stereoisomer thereof, or a combination thereof, and the tyrosine kinase inhibitor may be directly administered into a subject by any means such as oral, intravenous, intramuscular, buccal, transdermal, mucosal, intranasal, intratracheal, or subcutaneous administration. The compound, a pharmaceutically acceptable salt, solvate, or stereoisomer thereof, or a combination thereof, and the tyrosine kinase inhibitor may be systemically or locally administered singly or together with other pharmaceutically active compound.

The compound, a pharmaceutically acceptable salt, solvate, or stereoisomer thereof, or a combination thereof, and the tyrosine kinase inhibitor may be administered simultaneously, individually, or sequentially. For example, after administering dasatinib to a subject, the compound of Chemical Formula II, a pharmaceutically acceptable salt, solvate, or stereoisomer thereof, or a combination thereof may be administered to the subject.

A preferred administration dose of the compound, a pharmaceutically acceptable salt, solvate, or stereoisomer thereof, or a combination thereof, and tyrosine kinase inhibitor may differ depending on a patient's conditions and body weight, severity of the disease, drug formulation, administration route and period, but it may be properly selected by those skilled in the art. The administration dose may be, for example, within the range of about 0.001 mg/kg to about 100 mg/kg, about 0.01 mg/kg to about 10 mg/kg, or about 0.1 mg/kg to about 1 mg/kg per adult. The administration may be performed once a day, several times a day, twice or three times a week, once to four times a month, or once or twelve times a year.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the exemplary embodiments are merely described below, by referring to the figures, to explain aspects of the present description. As used herein, the term "and/or" includes any and all combinations of one or more of the associated listed items.

Hereinafter, the present invention will be described in more detail with reference to Examples. However, these Examples are for illustrative purposes only, and the invention is not intended to be limited by these Examples.

### Example 1. Test of CML therapeutic effect of combination of tyrosine kinase inhibitor and TEW-7197

### 1. Preparation of mouse model and CML stem cell

Scl/Tal1-tTA x TRE-BCR-ABL1 double transgenic (tg) mice was prepared by mating Scl/Tal1-tTA tg mouse (JAX^{®} mice, Stock No. 006209, Jackson Laboratory) and TRE-BCR-ABL1 transgenic mouse (JAX^{®} mice, Stock No. 006202, Jackson Laboratory). These mice was maintained while supplying water containing 20 mg/L of doxycycline (Sigma-Aldrich). 5 weeks after birth, water containing doxycycline was replaced by water containing no doxycycline (doxycycline-end) to induce expression of BCR-ABL1 oncogene. About 2 weeks to 5 weeks after the doxycycline end date, CML-like disease occured in the transgenic mice. These mice are SCL-tTA/BCR-ABL-tetO double positive mice, and designated as tetracycline(tet)-inducible "tg-CML affected mouse".

The primitive, long term (LT)-CML stem cells (CD150⁺CD135⁻CD48⁻c-Kit⁺Lineage⁻Sca-1⁺ cell) was separated from the tet-inducible tg-CML affected mice.

Meanwhile, hematopoietic stem cells of human BCR-ABL1-GFP gene-introduced mouse were transplanted to C57BL/6 (Sankyo-Lab Service, Japan) mouse to prepare a transduction/transplantation (TT)-CML affected mouse model (Naka et. al., Nature 2010; 463: 676-680). A CML-MPP (multipotent progenitor) fraction containing GFPBCR-ABL1-positive and GFPBCR-ABL1 T315I-positive c-Kit⁺Lineage⁻Sca-1⁺(KLS⁺) cells was separated from the TT-CML affected mouse (Naka et. al., Nature 2010; 463: 676-680). Further, human BCR-ABL1 T315I mutant-GFP gene-introduced mouse hematopoietic stem cells were transplanted to mice by the above described method to prepare TKI-resistant T315I TT-CML affected mice (Naka et. al., Nature 2010; 463: 676-680).

### 2. Preparation of drug

As a vehicle, 7 ml of 37%(v/v) gastric acid, 2.0 g of NaCl, 3.2 g of pepsin (Sigma-Aldrich), and distilled water was mixed to prepare 1000 ml of artificial gastric fluid.

As an administration drug, N-((4-([1,2,4]triazolo[1,5-a]pyridin-6-yl)-5-(6-methylpyridin-2-yl)-1H-imidazol-2-yl)methyl)-2-fluoroaniline (hereinbelow, referred to as 'TEW-7197') (Syngene, India) was dissolved in the vehicle to prepare 2 mg/ml of TEW-7197 solution. As a comparative control to TEW-7197, Ly2157299 (Selleck Chemicals) was dissolved in the vehicle to prepare a Ly2157299 solution.

Further, as a tyrosine kinase inhibitor (TKI), each of imatinib mesylate (Gleevec^{®}, Novartis), nilotinib (Tasigna^{®}, Novartis), dasatinib (Sprycel^{®}, Bristo-Myers Squibb), and ponatinib (Selleck Chemicals, AP24534) was dissolved in the vehicle to prepare drugs.

### 3. In vitro test of CML therapeutic effect of combination of tyrosine kinase inhibitor and TEW-7197

About 100,000 of mouse mesenchymal stem cell, OP-9 cell (ATCC^{®} CRL-2749) were cultured as a monolayer in a 24-well plate for about 1 day. 100,000 of LT-CML stem cells prepared in 1. were added to the cultured OP-9 cells to prepare a cell culture broth.

5 µM of TEW-7197 was added to the cell culture broth, and the cells were cultured under conditions of 3% oxygen and 37°C for about 1 day. Next day, 1 µM of imatinib mesylate, 1 µM of nilotinib, 1 µM of dasatinib, or 1 µM ponatinib was added to the cultured cells, respectively and the cells were cultured under conditions of 3% oxygen and 37°C for about 2 day. A co-culture period of LT-CML stem cells and OP-9 cells was total 3 days. As a control group, dimethyl sulfoxide (DMSO) was used instead of the drugs. Thereafter, the cell culture broths were washed with a phosphate buffer and cells were collected. The collected cells were cultured in methyl cellulose (Stem cell technologies, GFM3434) under conditions of 3% oxygen and 37°C for about 1 week, and colony formation of CML stem cells was measured in the cultured cells.

Colony formation of LT-CML stem cells cultured in the presence of TEW-7197, imatinib, nilotinib, dasatinib, or ponatinib alone, or a combination thereof is shown in FIG. 1. As shown in FIG. 1, a significant reduction in colony formation was observed in co-treatment of TEW-7197 and imatinib, nilotinib, dasatinib, or ponatinib, compared to single treatment of TEW-7197, imatinib, nilotinib, or dasatinib. In particular, a combination of TEW-7197 and dasatinib and a combination of TEW-7197 and ponatinib significantly inhibited colony formation. Therefore, it was confirmed that a combination of TEW-7197 and a tyrosine kinase inhibitor has an inhibitory effect on CML stem cells.

### 4. In vivo test of mouse survival rate by combination of tyrosine kinase inhibitor and TEW-7197

### (1) Effect of combination of imatinib and TEW-7197 in TT-CML-affected mouse

Cells were transplanted to the prepared TT-CML affected mouse as described in 1. and on day 8 post-transplantation, the vehicle or imatinib (200 mg/kg/ day) was orally administered. From day 15 to day 90 post-transplantation, vehicle; vehicle and TEW-7197 (2.5 mg/kg, every three days); vehicle and imatinib (200 mg/kg/day); imatinib (200 mg/kg/ day) and Ly2157299 (150 mg/kg, every three days), or imatinib (200 mg/kg/ day) and TEW-7197 (2.5 mg/kg, every three days) were administered.

After drug administration, mouse survival was monitored until day 125 post-transplantation. From this result, survival rate (%) of TT-CML affected mouse over time was calculated and the result is shown in FIG. 2A (- - - -: vehicle (n=23), - - - -: vehicle and TEW-7197 (n=24), bold line: vehicle and imatinib (n=24), - - -: imatinib and Ly2157299 (n=24), solid line: imatinib and TEW-7197 (n=24)).

As shown in FIG. 2A, the concurrent administration of imatinib and TEW-7197 increased survival rate of TT-CML-affected mouse, compared to single administration of imatinib or TEW-7197, and concurrent administration of imatinib and Ly2157299. Therefore, the combination of TEW-7197 and imatinib exhibits a prophylactic or therapeutic effect on CML in vivo.

### (2) Effect of combination of dasatinib and TEW-7197 in tg-CML affected mouse

To examine *in vivo* therapeutic effects of combination of TEW-7197 and dasatinib in tg-CML affected mouse, tg-CML affected mice were prepared as described in 1.

Water for feeding the tg-CML affected mice was replaced by water containing no doxycycline. The tg-CML affected mice were orally administered with dasatinib at a dose of 5 mg/kg/day once a day for 1 day to 36 days after the end of doxycycline treatment. Additionally, dasatinib alone, or dasatinib and TEW-7197 (2.5 mg/kg/day) was/were orally administered every two days for 8 days to 36 days after the end of doxycycline treatment. As a control group, an vehicle containing no drug was used.

After the end of doxycycline treatment, survival rate(%) of tg-CML affected mouse over time was calculated and the result is shown in FIG. 2B (dotted line: vehicle (n=20), bold line: dasatinib alone (n=12), solid line: dasatinib and TEW-7197(n=11)).

As shown in FIG. 2B, the concurrent administration of dasatinib and TEW-7197 increased survival rate of tg-CML affected mouse, compared to the control group and single administration of dasatinib. Therefore, it was confirmed that the combination of TEW-7197 and dasatinib exhibits a prophylactic or therapeutic effect on CML in vivo.

### (3) Effect of combination of ponatinib and TEW-7197 in TKI-resistant T315I TT-CML affected mouse

Cells were transplanted to the prepared TKI-resistant T315I TT-CML affected mouse as described in 1., and on day 8 post-transplantation, the vehicle or ponatinib (15 mg/kg/day) was orally administered. From day 15 to day 60 post-transplantation, vehicle; vehicle and TEW-7197 (2.5 mg/kg, every three days); vehicle and ponatinib (15 mg/kg/day); or ponatinib (15 mg/kg/ day) and TEW-7197 (2.5 mg/kg, every three days) were administered.

After drug administration, mouse survival was monitored until day 100 post-transplantation. From this result, survival rate (%) of TKI-resistant T315I TT-CML affected mouse over time was calculated and the result is shown in FIG. 2C (- - - -: vehicle (n=23), solid line: vehicle and TEW-7197 (n=37), bold line: vehicle and ponatinib (n=32), - - -: ponatinib and TEW-7197 (n=32)).

As shown in FIG. 2C, the concurrent administration of ponatinib and TEW-7197 increased survival rate of TKI-resistant T315I TT-CML affected mouse, compared to single administration of ponatinib or TEW-7197. Therefore, it was confirmed that the combination of TEW-7197 and ponatinib exhibits a prophylactic or therapeutic effect on tyrosine kinase-resistant CML in vivo.

### 5. In vivo effect of combination of tyrosine kinase inhibitor and TEW-7197

### (1) CML therapeutic effect of combination of dasatinib and TEW-7197 in TT-CML-affected mouse

Dasatinib (5 mg/kg/day), TEW-7197 (2.5 mg/kg/day, every three days), or dasatinib (5 mg/kg/day) and TEW-7197 (2.5 mg/kg/day, every three days) were orally administered for about 30 days to the prepared TT-CML affected mouse as described in 1.

The number of leukocyte in the peripheral blood of the drug-administered TT-CML affected mouse is shown in FIG. 3A (n=5, NS: not significant), a photograph of the spleen thereof is shown in FIG. 3B (bar: 10 mm), and the weight of the spleen thereof is shown in FIG. 3C (n=3). Further, percentages (%) of T cell (detected using anti-CD4 antibody and anti-CD8 antibody), B cell (detected using anti-B220 antibody), and bone marrow cell (detected using anti-Mac1 antibody and anti-Gr-1 antibody) among the total BCR-ABL1-GFP⁺ cells in the peripheral blood of the TEW-7197-administered TT-CML affected mouse are shown in FIG. 3D (n=3, NS: not significant).

As shown in FIGS. 3A though 3C, the number of leukocyte in the peripheral blood was increased and splenomegaly was promoted in the TT-CML affected mouse administered with TEW-7197, compared to the control group administered with the vehicle. However, the increase in the number of leukocyte in the peripheral blood and splenomegaly were inhibited in the TT-CML affected mouse administered with dasatinib and TEW-7197. As shown in FIG. 3D, administration of TEW-7197 did not affect differentiation of bone marrow cells among the BCR-ABL1-GFP⁺ cells. Therefore, it was confirmed that dasatinib inhibits proliferation of CML cells of which differentiation is induced by TEW-7197 in vivo.

After cell transplantation, dasatinib (5 mg/kg/day), TEW-7197 (2.5 mg/kg/day, every three days), or dasatinib (5 mg/kg/day) and TEW-7197 (2.5 mg/kg/day, every three days) were orally administered for 30 days to the TT-CML affected mouse. GFP/BCR-ABL⁺ c-Kit⁺Lineage⁻Sca-1⁺(KLS⁺) cells (bold box) and c-Kit⁺Lineage⁻Sca-1⁻(KLS⁻) cells (dotted box) in the peripheral blood were analyzed by flow cytometry, and the results are shown in FIG. 4A. Cells were gated on GFP⁺ and Lineage⁻, and the percentages (%) of GFP/BCR-ABL⁺ KLS⁻ cells and KLS⁺ cells among the total GFP/BCR-ABL⁺ cells of TT-CML affected mouse are shown in FIGS. 4B and 4C, respectively (n=3, NS: not significant).

As shown in FIGS. 4A through 4C, and the percentages (%) of KLS⁻ cells and KLS⁺ cells among the BCR-ABL1-GFP⁺ CML cells were remarkably decreased in the TT-CML affected mouse administered with TEW-7197. Single administration of dasatinib decreased the percentage of CML KLS⁻ cells, but concurrent administration of dasatinib and TEW-7197 further decreased the percentage of CML KLS⁻ cells, indicating that TEW-7197 inhibits self-renewal ability of primitive CML-MPP, but did not inhibit proliferation of differentiated CML cells. Therefore, concurrent administration of dasatinib and TEW-7197 eliminates TKI-insensitive CML-MPP to provide a therapeutic effect for CML patients.

### (2) TKI-resistant CML therapeutic effect of combination of ponatinib and TEW-7197 in TKI-resistant T315I TT-CML affected mouse

Ponatinib (15 mg/kg/day), TEW-7197 (2.5 mg/kg/day, every three days), or ponatinib (15 mg/kg/day) and TEW-7197 (2.5 mg/kg/day, every three days) were orally administered for 30 days to the prepared TKI-resistant T315I TT-CML affected mouse as described in 1. T315I BCR-ABL-GFP⁺ KLS⁺ cells (bold box) and KLS⁻ cells (dotted box) in the peripheral blood were analyzed by flow cytometry, and the results are shown in FIG. 5A. Cells were gated on GFP⁺ and Lineage⁻, and the percentages (%) of T315I BCR-ABL-GFP⁺ KLS⁻ cells and KLS⁺ cells among the total T315I BCR-ABL-GFP⁺ cells of TKI-resistant T315I TT-CML affected mouse are shown in FIGS. 5B and 5C, respectively (n=3, NS: not significant).

As shown in FIGS. 5A through 5C, the percentage (%) of T315I BCR-ABL-GFP⁺ KLS⁺ cells was remarkably decreased in the TKI-resistant T315I TT-CML affected mouse administered with TEW-7197. Concurrent administration of ponatinib and TEW-7197 further decreased the percentage of T315I BCR-ABL-GFP⁺ KLS⁺ cells. Therefore, concurrent administration of ponatinib and TEW-7197 as well as single administration of TEW-7197 effectively blocks self-renewal ability of T315I-CML KLS⁺ cells, thereby providing a therapeutic effect for CML patients.

### 6. Inhibitory effect of combination of dasatinib and TEW-7197 on colony formation of human CML-initiating cells in vitro

Bone marrow mononuclear cells (Allcells, Cat. No. 06-255, 06-620, and 147742, CA) of three patients with chronic CML were prepared.

Bone marrow mononuclear cells were stained with anti-CD34(8G12) antibody (BD Biosciences), anti-CD38(HIT2) antibody (BD Biosciences), anti-CD3(SK7) antibody (BD Biosciences), anti-CD16(3G8) antibody (BD Biosciences), anti-CD19(SJ25C1) antibody (BD Biosciences), anti-CD20(L27) antibody (BD Biosciences), anti-CD14(MfP9) antibody (BD Biosciences), and anti-CD56(NCAM16.2) antibody (BD Biosciences). A mouse antibody cocktail recognizing CD3, CD16, CD19, CD20, CD14 and CD56 was used to identify Lineage⁻ (Lin⁻) cells, and CD34⁺CD38⁻Lin⁻ cells were separated. CD34⁺CD38⁻Lin⁻ cells were co-cultured with OP-9 stromal cells (ATCC^{®} CRL-2749) in the presence of TEW-7197 alone, dasatinib alone, or TEW-7197 and dasatinib under conditions of 3% oxygen and 37°C. A control group was prepared in the same manner, except that DMSO was used. Cells were harvested and washed with PBS, and then cultured in methyl cellulose (Stem cell technologies, GFM3434) to measure colony formation of human CML-initiating cells. Colony formations of human CML-initiating cells of the three patients thus measured are shown in FIGS. 6A through 6C.

As shown in FIGS. 6A through 6C, TEW-7197 significantly inhibited colony formation of human CML-initiating cells in vitro. Further, co-treatment of TEW-7197 and dasatinib significantly inhibited colony formation of human CML-initiating cells, compared to single treatment of dasatinib. Therefore, it was confirmed that combination of TKI and TEW-7197 eliminates primitive CML-initiating cells in human CML patients.

It should be understood that exemplary embodiments described herein should be considered in a descriptive sense only and not for purposes of limitation. Descriptions of features or aspects within each exemplary embodiment should typically be considered as available for other similar features or aspects in other exemplary embodiments.

## Claims

1. A pharmaceutical composition for use in a method of preventing or treating chronic myeloid leukemia, the composition comprising a compound of the following Chemical Formula II, a pharmaceutically acceptable salt, solvate, or stereoisomer thereof, or a combination thereof and a tyrosine kinase inhibitor: 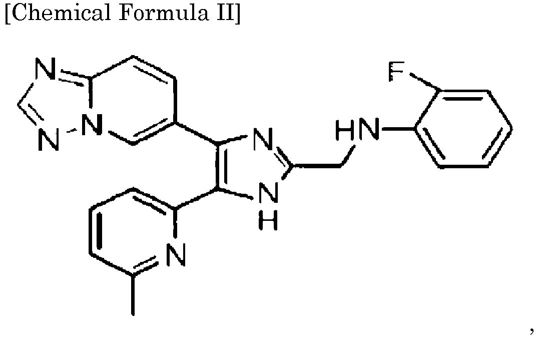 wherein the tyrosine kinase inhibitor is imatinib, dasatinib, nilotinib, bosutinib, ponatinib, or a combination thereof.

2. The pharmaceutical composition for use of claim 1, wherein the chronic myeloid leukemia is tyrosine kinase inhibitor-resistant.

3. The pharmaceutical composition for use of claim 2, wherein the tyrosine kinase inhibitor-resistance is caused by a BCR-ABL fusion protein having a mutation of replacing a tyrosine residue by an isoleucine residue at position 315.

4. The pharmaceutical composition for use of claim 1, wherein the pharmaceutical composition is a single composition or individual compositions.

5. The pharmaceutical composition for use of claim 1, wherein the compound of Chemical Formula II, the pharmaceutically acceptable salt, solvate, or stereoisomer thereof, or the combination thereof; and the tyrosine kinase inhibitor is suitable to be administered simultaneously, individually or sequentially.

6. The pharmaceutical composition for use of claim 1, wherein the compound of Chemical Formula II, the pharmaceutically acceptable salt, solvate, or stereoisomer thereof, or a combination thereof is suitable to be administered to the subject, after administering the tyrosine kinase inhibitor to the subject.

## Patentansprüche

1. Pharmazeutische Zusammensetzung zur Verwendung in einem Verfahren zum Vorbeugen oder Behandeln chronischer myeloider Leukämie, die Zusammensetzung umfassend eine Verbindung der folgenden chemischen Formel II, ein pharmazeutisch akzeptables Salz, Solvat, oder Stereoisomer davon, oder eine Kombination davon und einen Tyrosinkinase-Inhibitor: 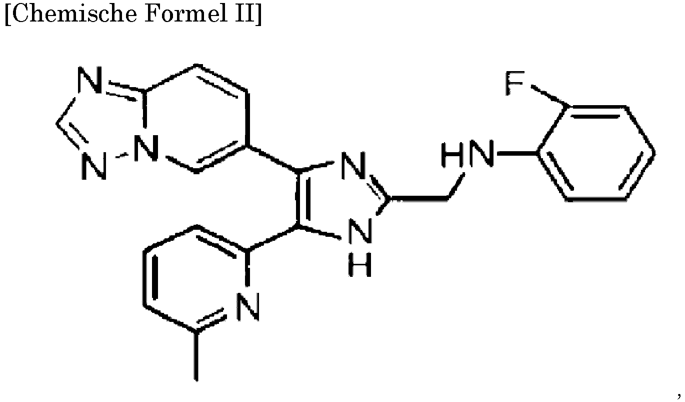 wobei der Tyrosinkinase-Inhibitor Imatinib, Dasatinib, Nilotinib, Bosutinib, Ponatinib, oder eine Kombination davon ist.

2. Pharmazeutische Zusammensetzung zur Verwendung nach Anspruch 1, wobei die chronische myeloide Leukämie Tyrosinkinase-Inhibitor-resistent ist.

3. Pharmazeutische Zusammensetzung zur Verwendung nach Anspruch 2, wobei die Tyrosinkinase-Inhibitor Resistenz durch ein BCR-ABL Fusionsprotein mit einer Mutation, ersetzend einen Tyrosin-Rest durch ein Isoleucin-Rest an Position 315, verursacht ist.

4. Pharmazeutische Zusammensetzung zur Verwendung nach Anspruch 1, wobei die pharmazeutische Zusammensetzung eine einzelne Zusammensetzung oder individuelle Zusammensetzungen sind.

5. Pharmazeutische Zusammensetzung zur Verwendung nach Anspruch 1, wobei die Verbindung nach chemischer Formel II, das pharmazeutisch akzeptable Salz, Solvat, oder Stereoisomer davon, oder die Kombination davon; und der Tyrosinkinase-Inhibitor geeignet ist, gleichzeitig, individuell oder nacheinander verabreicht zu werden.

6. Pharmazeutische Zusammensetzung zur Verwendung nach Anspruch 1, wobei die Verbindung nach chemischer Formel II, das pharmazeutisch akzeptable Salz, Solvat, oder Stereoisomer davon, oder eine Kombination davon geeignet ist, nach Verabreichen des Tyrosinkinase-Inhibitors an das Subjekt, an das Subjekt verabreicht zu werden.

## Revendications

1. Une composition pharmaceutique destinée à être utilisée dans une méthode de prévention ou de traitement de la leucémie myéloïde chronique, la composition comprenant un composé de formule chimique II suivante, un de ses sels, un de ses solvates ou un de ces stéréoisomères pharmaceutiquement acceptables, ou une combinaison de ceux-ci et un inhibiteur de tyrosine kinase : 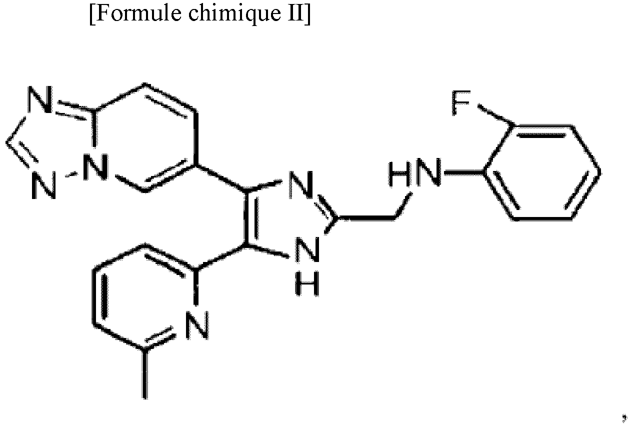 dans lequel l'inhibiteur de tyrosine kinase est l'imatinib, le dasatinib, le nilotinib, le bosutinib, le ponatinib ou une combinaison de ceux-ci.

2. La composition pharmaceutique pour une utilisation selon la revendication 1, dans laquelle la leucémie myéloïde chronique est résistante aux inhibiteurs de la tyrosine kinase.

3. La composition pharmaceutique pour utilisation selon la revendication 2, dans laquelle la résistance à l'inhibiteur de la tyrosine kinase est provoquée par une protéine de fusion BCR-ABL présentant une mutation consistant en le remplacement d'un résidu tyrosine par un résidu isoleucine en position 315.

4. La composition pharmaceutique pour une utilisation selon la revendication 1, dans laquelle la composition pharmaceutique est une composition unique ou des compositions individuelles.

5. La composition pharmaceutique pour une utilisation selon la revendication 1, dans laquelle le composé de formule chimique II, le sel, le solvate ou le stéréoisomère pharmaceutiquement acceptable de celui-ci, ou leur combinaison, et l'inhibiteur de tyrosine kinase est adapté pour être administré simultanément, individuellement ou séquentiellement.

6. La composition pharmaceutique destinée à être utilisée selon la revendication 1, dans laquelle le composé de formule chimique II, le sel, le solvate ou le stéréoisomère pharmaceutiquement acceptable de celui, ou une combinaison de ceux-ci, conviennent pour une administration au sujet après que l'inhibiteur de tyrosine kinase lui a été administré.
